# EUROPÄISCHE PATENTSCHRIFT

(11) **EP 0 488 987 B1**
(45) Veröffentlichungstag und Bekanntmachung des Hinweises auf die Patenterteilung: **31.01.1996**
(21) Anmeldenummer: 91890231.3
(22) Anmeldetag: 01.10.1991
(51) Int. Cl.: A61B 5/11

(54) **Verfahren zur Darstellung beweglicher Körper**
Method for representing moving bodies
Méthode de représentation de corps mobiles

(30) Priorität: 26.11.1990 AT 2397/90; 26.11.1990 AT 2398/90
(43) Veröffentlichungstag der Anmeldung: 03.06.1992
(73) Patentinhaber: Truppe, Michael, Dr., A-1130 Wien (AT)
(72) Erfinder: Truppe, Michael, Dr., A-1130 Wien (AT)
(74) Vertreter: Babeluk, Michael, Dipl.-Ing.

(56) Entgegenhaltungen:
- EP-A- 0 119 660
- FR-A- 2 545 349
- PROCEEDINGS OF THE ANNUAL INTERNATIONAL CONFERENCE OF THE IEEE ENGINEERING IN MEDICINE AND BIOLOGY SOCIETY. Bd. 11, 1989, US, Seiten 1445-1446; MAEKAWA ET AL.:"Measurement of the upper limb motion by a magnetic sensor and its application to kinesiological studies."

## Beschreibung

Die Erfindung betrifft ein Verfahren zur Darstellung beweglicher Körper.

Es ist in der Technik und in der Medizin oftmals erforderlich, die optische Darstellung von Objekten mit zusätzlichen Informationen anzureichern oder verschiedene Darstellungen eines Gegenstandes miteinander zu kombinieren.

So ist es beispielsweise bei der Durchführung bestimmter Operationen am menschlichen Körper üblich und notwendig, präoperative Bilder mit intraoperativen Bildern zu vergleichen. Bei diesen Bildern kann es sich um zweidimensionale Röntgenaufnahmen, Tomographien, Ultraschalldarstellungen oder Kernspintomographien handeln.

Um zwei solcher Darstellungen in Übereinstimmung zu bringen ist es nun möglich, eine gewisse Anzahl anatomisch charakteristischer Punkte zu identifizieren und deren Koordinaten in den beiden Darstellungen festzustellen. Danach kann man die sechs Parameter einer Festkörpertransformation, nämlich drei translatorische und drei rotatorische Parameter sowie gegebenenfalls einen Skalierungsfaktor berechnen. Damit ist es möglich, eine der beiden Darstellungen so zu drehen und zu verschieben, daß beide Bilder aus der selben Perspektive und im selben Maßstab angezeigt werden können. Dieser Prozeß wird als "Matching" bezeichnet.

Die Identifikation der charakteristischen Punkte ist bei diesem Verfahren aufwendig und fehleranfällig. Außerdem können damit stets nur zwei Einzelbilder ex post verglichen und dargestellt werden.

Um nun das Matching zu vereinfachen wurde vorgeschlagen, am zu untersuchenden Objekt gewisse Markierungen anzubringen, die in beiden Darstellungen sichtbar und leicht identifizierbar sind. Dies können Sonden aus einem passenden Material sein, deren Position auf Röntgenaufnahmen leicht feststellbar ist. Im Fall von optischen Darstellungen kann es sich um Farbmarkierungen auf der Hautoberfläche handeln. Auch damit ist nur eine ex post Darstellung zweier Einzelbilder möglich.

Um nun eine optische Darstellung eines Gegenstandes mit anderen Darstellungen, bei denen es sich wie oben erwähnten zweidimensionale Röntgenaufnahmen, Tomographien, Ultraschalldarstellungen oder Kernspintomographien oder dgl. handeln kann, zu kombinieren, ist es im Prinzip möglich, zunächst ein dreidimensionales Modell der Oberfläche des Körpers zu schaffen und abzuspeichern. Dieses Modell kann nun in der oben erläuterten Weise etwa mit den Daten einer Kernspintomographie gematcht werden. Danach ist es möglich, den Körper aus einer beliebigen Perspektive optisch darzustellen und gleichzeitig Daten aus der Tomographie passend einzublenden. Die optische Darstellung wird dabei aus dem Modell errechnet.

Ein solches Verfahren ist sehr aufwendig, da sowohl ein großer Speicherplatzbedarf besteht als auch eine enorme Rechnerleistung erforderlich ist. Außerdem ist auch hier keine Darstellung in Echtzeit möglich.

Aus der AT-B 384 544 ist ein Verfahren zur Positionsbestimmung von Körperteilen bekannt. Dabei wird mittels Ultraschallsensoren die jeweilige Lage bestimmt, um die Gelenksbeweglichkeit festzustellen. Es handelt sich dabei jedoch um ein rein rechnerisches Verfahren, das keine bildliche Darstellung erlaubt und deshalb unanschaulich ist.

Der gleiche Nachteil trifft auch für die in der DE-C 34 06 179 beschriebene Vorrichtung zu.

Die US-A 4,197,855 beschreibt eine Vorrichtung zur Messung der Bewegung des menschlichen Unterkiefers, bei der ein winkelförmiger Permanentmagnet zur Bestimmung der räumlichen Position dient. Auch hier ist eine bildgebende Weiterverarbeitung nicht vorgesehen.

Aus der DE-A 38 07 578 ist es bekannt, einzelne Punkte auf einem menschlichen Kopf mit Hilfe von Videokameras zu vermessen. Die zu vermessenden Punkte werden dabei gemeinsam mit am Kopf angebrachten Bezugspunkten in einem stehenden Bild aufgenommen und elektronisch erfaßt. Eine optische Darstellung ist nicht einmal für stehende Einzelbilder vorgesehen. Umso weniger ist eine Darstellung in Echtzeit nahegelegt.

FR-A-2 545 349 beschreibt eine Verfahren, mit dem zwei Schnittdarstellung des Körpers in Übereinstimmung gebracht werden können, wie z.B. ein Röntgenbild und eine Infrarotdarstellung.

EP-A-119660 beschreibt ein System zur Untersuchung des Skeletts, mit den Merkmale des Oberbegriffs der Ansprüche.

Aufgabe der Erfindung ist es, diese Nachteile zu vermeiden und ein Verfahren zu schaffen, das es in einfacher Weise ermöglicht, optische Darstellungen in anschaulicher Weise mit anderen Daten zu kombinieren in übersichtlicher Weise darzustellen.

Beim erfindungsgemäßen Verfahren ist vorgesehen, daß eine optische Darstellung des Körpers und ein dem Körper zugeordnetes Datenfeld gleichzeitig oder alternativ aus der selben Perspektive und im selben Maßstab in Echtzeit dargestellt werden. Dies bedeutet, daß sich der Körper im Aufnahmebereich frei bewegen kann. Dabei wird er in Echtzeit - also gleichzeitig - auf einer Anzeigevorrichtung dargestellt. Ein gespeichertes Datenfeld wird mit der Bewegung des Körpers mitgeführt und kann ebenfalls dargestellt werden. Auf diese Weise wird ein großes Maß an Anschaulichkeit erreicht.

Das erfindungsgemäße Verfahren ist definiert in Anspruch 1.

Es ist möglich, daß als Datenfeld die Position anatomisch charakteristischer Punkte, eine Röntgentomographie, eine Kernspintomograhpie oder eine Ultraschalldarstellung verwendet wird.

Die Position von anatomisch charakteristischen Punkten kann etwa durch einen 3D- Digitizerstylus, also einen Magnetgriffel, erfaßt werden. Dabei wird mit der Spitze des Stylus der betreffende Punkt markiert und gleichzeitig durch Betätigung der Eingabetaste die Bestimmung der genauen Position ausgelöst. Es ist klar, daß dadurch nur an der Körperoberfläche liegende Punkte markiert werden können.

Wenn Punkte, die im Körperinneren liegen, wie zum Beispiel charakteristische Punkte von Knochen, als Datenpunkte herangezogen werden sollen, so kann man diese etwa durch stereophotometrische Vermessung auf Röntgenaufnahmen ermitteln. Ein Anwendungsfall hierfür ist die Vermessung von Gelenksbewegungen. Dabei werden zunächst zwei oder mehr Röntgenaufnahmen der entsprechenden Gliedmaße mit auf der Hautoberfläche angebrachten Sensoren hergestellt. Vorzugsweise sind die Bildebenen dieser Aufnahmen zueinander rechtwinkelig. dadurch ist es möglich, die Koordinaten von charakteristischen Punkten zu bestimmen.

In einer bevorzugten Variante des Verfahrens ist vorgesehen, daß zur Erfassung der Lage des Körpers ein 3D-Sensor, vorzugsweise ein Magnet-Sensor, fest mit dem Körper verbunden wird. Die laufende Ermittlung der Lage des Körpers ist damit in einfacher Weise möglich.

Das Datenfeld braucht sich jedoch nicht auf die Position von Punkten des Körpers selbst zu beschränken. Als Datenfeld kann ein Röntgenbild verwendet werden, dessen Position zum Körper dadurch bestimmt wird, daß bei der Röntgenaufnahme ein 3D-Sensor fest mit dem Körper und ein weiterer solcher Sensor mit der Röntgenkassette verbunden ist. Das gewöhnliche zweidimensionale Röntgenbild wird einer Ebene außerhalb des Körpers zugeordnet, z.B. der Ebene, in der sich bei der Aufnahme der Röntgenfilm befunden hat. Dieses Röntgenbild stellt dann das Datenfeld dar und es ist möglich, den Körper eines Patienten mit dessen Röntgenbild so darzustellen, als ob das Röntgenbild fest mit ihm verbunden wäre.

In analoger Weise kann auch als Datenfeld eine Ultraschalldarstellung verwendet werden, deren Position zum Körper dadurch bestimmt wird, daß bei der Beschallung ein 3D-Sensor, vorzugsweise ein Magnet-Sensor, fest mit dem Körper und ein weiterer solcher Sensor mit dem Ultraschallkopf verbunden ist.

Bei einer bevorzugten Ausführungsvariante der Erfindung ist vorgesehen, daß zur Erfassung der Lage des Körpers ein 3D-Sensor, vorzugsweise ein Magnet-Sensor, fest mit dem Körper verbunden wird. Ein solcher Sensor besteht aus einem kleinen Magneten, der nicht nur die Ermittlung seiner räumlichen Position, sondern auch die Bestimmung seiner Winkellage erlaubt.

Es ist möglich, daß das optische Bild mit der Darstellung des Datenfeldes am Bildschirm überlagert wird. Dabei ist es möglich, daß dieses Datenfeld in die optische Darstellung eingeblendet wird. Es ist jedoch auch möglich, daß zwischen optischer Darstellung und der Datendarstellung hin und her geschalten werden kann. Dem Anwender sind dadurch vielfältige Möglichkeiten geboten, sich sowohl eine anschauliche Darstellung zu verschaffen, als auch eine optimale Grundlage für eine exakte Vermessung von Körperteilen zu erhalten.

Besonders günstig ist es, wenn das optische Bild und die Darstellung des Datenfeldes am Bildschirm in unterschiedlichen Fenstern angezeigt werden. Diese Fenster können vom Benutzer in an sich bekannter Weise verschoben, übereinandergelegt, gezoomt oder ausgeblendet werden.

In einer besonders begünstigten Ausführungsvariante des erfindungsgemäßen Verfahrens ist vorgesehen, daß ein Teil des darstellbaren Datenfeldes aus einer willkürlich wählbaren gedachten Achse besteht und daß diese Achse durch folgende Schritte gewonnen wird:
- Darstellung des Körpers als stehendes Bild in mindestens zwei Stellungen;
- Bereitstellung der Möglichkeit durch den Benutzer das Bild der Achse in die stehenden Bilder einzuzeichnen;
- Berechnung der räumlichen Lage der Achse;
- gemeinsame Darstellung der Achse mit dem in Echtzeit beweglich dargestellten Körper.

Soll beispielsweise in das Bild eines menschlichen Oberschenkels die Achse des Femurs eingeblendet werden, so wird folgendermaßen vorgegangen: Zunächst wird der Lagesensor auf eine durch Weichteilbewegungen möglichst wenig beeinflußte Hautpartie aufgeklebt. Dann werden mit der Videokamera Einzelbilder aufgenommen und gleichzeitig oder hintereinander am Bildschirm angezeigt. Durch den Lagesensor ist dabei die Position des Körperteil im Zeitpunkt der Aufnahme des Bildes bekannt. Der Benutzer kann dann etwa mit Hilfe einer Maus in jedes Einzelbild eine Achse einzeichnen. Für den Rechner stellt dies die Projektion einer im dreidimensionalen Raum befindlichen Achse auf die Bildebene dar. Wenn diese Achse in zwei verschiedenen Darstellungen, beispielsweise in einer Vorderansicht und einer Seitenansicht erfolgt ist, kann damit die räumliche Position bestimmt werden.

Es hat sich in diesem Zusammenhang als günstig herausgestellt, wenn etwa vier Einzelbilder nebeneinander in jeweils einem Fenster dargestellt werden. Der Benutzer jedes dieser Bilder zum Eintragen oder Ändern der Achse benützen. Sobald die räumliche Lage der Achse bestimmbar ist, wird diese auch für die übrigen Bilder berechnet und angezeigt. Somit ist eine genaue Kontrolle der Eingabe möglich.

In gleicher Weise ist es möglich, daß ein Teil des darstellbaren Datenfeldes aus einem willkürlich wählbaren Koordinatensystem besteht, dessen Achsen in der oben definierten Art gewonnen werden. Nach der räumlichen Festlegung einer Achse eines orthogonalen Koordinatensystems braucht lediglich eine weitere Achse in einem Bild eingetragen werden, um die Lage zu definieren.

Nach der Eingabe berechnet das Programm die Lage der Achse bzw. der Achsen in Bezug auf den Lagesensor und ist damit in der Lage, in jede Darstellung des Körperteils diese Achse einzublenden, sodaß sich diese mit dem Körperteil mitbewegt.

Die eingeblendete Achse, bzw. das Koordinatensystem kann an sich schon das Datenfeld im Sinne der Erfindung darstellen oder zusätzlich zu einer Röntgentomographie o. dgl. angezeigt werden.

Weiters betrifft die Erfindung eine Vorrichtung zur Darstellung beweglicher Körper. Erfindungsgemäß besteht diese Vorrichtung aus:
- einer Kamera;
- einem Monitor;
- einem Lagesensor, der mit dem darzustellenden Körper fest verbunden ist und die Bestimmung der jeweiligen Lage des Körpers ermöglicht;
- Mitteln, die die gleichzeitige oder alternative Darstellung des optischen Bildes und des Datenfeldes am Monitor ermöglichen.

Bei diesen letztgenannten Mitteln handelt es sich im allgemeinen um einen Computer, der die erforderlichen Berechnungen durchführt. Bei diesen Berechnungen ist der räumliche Abstand zwischen dem Brennpunkt der Kameraoptik und dem Ausgangspunkt des für den Lagesensor verwendeten Feldes, also etwa des von einem Magnetfeldemittor erzeugten magnetischen Feldes zu berücksichtigen. Weiters geht die relative Lage der Punkte des Datenfeldes in Bezug auf den Lagesensor in die Berechnung ein. An Stelle eines Computers kann jedoch auch ein entsprechend programmierter Mikroprozessor diese Berechnungen durchführen.

Eine besonders einfache und leicht realisierbare Ausführungsvariante der Erfindung ist dadurch gegeben, daß als Monitor der Bildschirm eines Personal Computers eingesetzt wird, wobei die Kamera am Videoeingang angeschlossen ist und das Datenfeld über den Personal Computer in einen weiten Eingang eingespielt wird. Dabei wird der aus der Aufnahme der optischen Bilder stammende Datenstrom vollständig am Computer vorbeigelenkt und belastet dessen Verarbeitungskapazität überhaupt nicht. Dadurch steht die gesamte Rechenleistung für die zusätzliche Darstellung des Datenfeldes zur Verfügung.

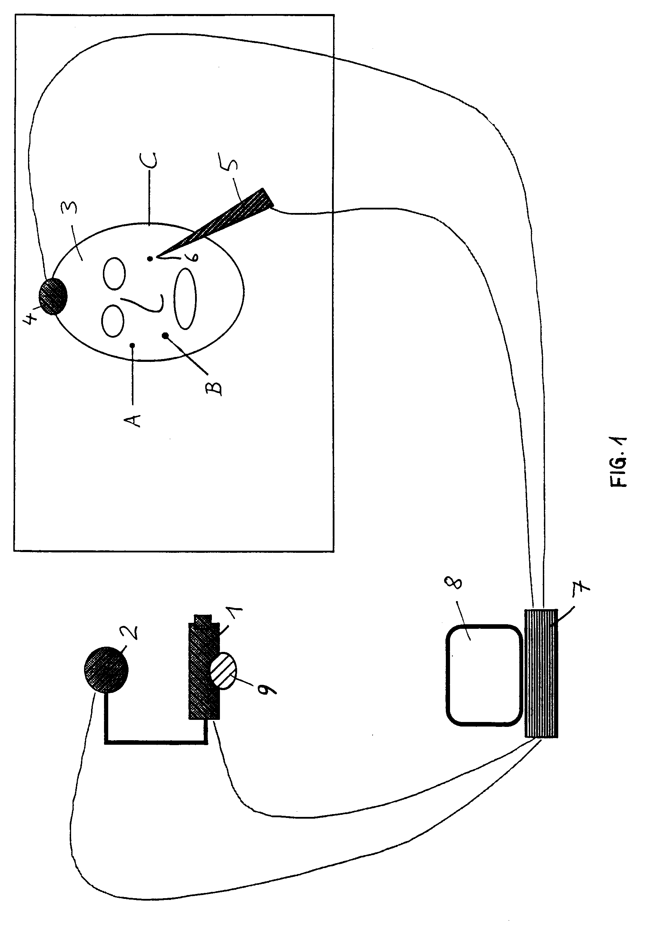

Im folgenden wird die Erfindung anhand eines in der Figur dargestellten Ausführungsbeispieles näher erläutert.

Eine Videokamera 1 ist fest mit einem Magnetfeldemittor 2 verbunden. Dieser Magnetfeldemittor 2 besteht im wesentlichen aus einer Spule, die an einen elektrischen Stromkreis angeschlossen ist und ein magnetisches Feld erzeugt. Am Kopf 3 des Patienten ist ein Magnetsensor 4 angebracht. Dieser Magnetsensor besteht ebenfalls aus Spulen, in denen durch das vom Magnetfeldemittor 2 erzeugte Feld Ströme induziert werden. Durch Messung dieser Ströme kann man die Position des Magnetsensors 4 auf etwa einen Millimeter genau bestimmen.

Mit einem Magnetgriffel 5 werden drei Meßpunkte A, B und C markiert. Auch im Magnetgriffel 5 sind wie beim Magnetsensor 4 Spulen zur Lageerkennung vorgesehen. Die Markierung erfolgt in der Weise, daß die Spitze 6 des Griffels an den entsprechenden Punkt gebracht wird und dann eine Auslösetaste betätigt wird. Der Computer 7 speichert die momentane Position der Spitze 6 als Datenpunkt. Auf dem Monitor 8 des Computers 7 wird der Kopf 3 zusammen mit den Datenpunkten A, B und C dargestellt. Dabei ist es möglich diese Datenpunkte auch dann darzustellen, wenn sie als reale Punkte nicht sichtbar wären, weil sie sich gerade auf der der Kamera abgewandten Seite des Kopfes befinden. Auch die Position anderer verdeckter Punkte ist dabei darstellbar. So kann mit dem Griffel 5 die Position eine bestimmten Zahnes markiert werden, der dann auch bei geschlossenem Mund lokalisiert werden kann. Falls sich dieser Zahn auf der unteren Zahnreihe befindet, muß jedoch die Stellung des Unterkiefers zusätzlich erfaßt werden. Dies ist durch die Anbringung eines nicht dargestellten weiteren Sensors möglich.

Wenn an der Kamera 1 ein zusätzlicher Magnetsensor 9 angebracht ist, so kann eine starre Kopplung zwischen der Kamera 1 und dem Magnetfeldemittor 2 entfallen. Der Computer 7 berücksichtigt dann die variable Versetzung zwischen Kamera 1 und Magnetfeldemittor 2.

## Patentansprüche

1. Verfahren zur Darstellung beweglicher Körper, bei dem eine optische Darstellung des Körpers und ein dem Körper zugeordnetes Datenfeld dargestellt werden, mit folgenden Schritten:
• Bereitstellung eines optischen Abbildungssystems bestehend aus Kamera und Monitor;
• Zuordnung eines räumlichen Datenfeldes zu dem in einer bestimmten Lage befindlichen Körper; indem die gedachten Koordinatensysteme des Körpers und des Datenfeldes miteinander so in Beziehung gebracht werden, daß eine Übereinstimmung erzielt wird;
gekennzeichnet durch:
• fortgesetzte Erfassung der räumlichen Position des Körpers, umfassend sowohl die translatorische als auch die rotatorische Verschiebung;
• fortgesetzte Berechnung einer Darstellung des Datenfeldes, die jeweils der Lage des Körpers entspricht;
• gleichzeitige oder alternative Darstellung des optischen Bildes und des Datenfeldes am Monitor so, daß die optische Darstellung des Körpers und das dem Körper zugeordnete Datenfeld gleichzeitig oder alternativ aus der selben Perspektive und im selben Maßstab in Echtzeit dargestellt werden.

2. Verfahren zur Darstellung eines menschlichen oder tierischen Körpers nach Anspruch 1, dadurch gekennzeichnet, daß als Datenfeld die Position anatomisch charakteristischer Punkte, oder eine Darstellung eines bildgebenden Verfahrens, wie etwa eine Röntgentomographie, eine Kernspintomographie oder eine Ultraschalldarstellung verwendet wird.

3. Verfahren nach einem der Ansprüche 1 oder 2, dadurch gekennzeichnet, daß zur Erfassung der Lage des Körpers ein 3D-Sensor, vorzugsweise ein Magnet-Sensor, fest mit dem Körper verbunden wird.

4. Verfahren nach Anspruch 3, dadurch gekennzeichnet, daß als Datenfeld ein Röntgenbild verwendet wird, dessen Position zum Körper dadurch bestimmt wird, daß bei der Röntgenaufnahme ein 3D-Sensor fest mit dem Körper und ein weiterer solcher Sensor mit der Röntgenkassette verbunden ist.

5. Verfahren nach Anspruch 3, dadurch gekennzeichnet, daß als Datenfeld eine Ultraschalldarstellung verwendet wird, deren Position zum Körper dadurch bestimmt wird, daß bei der Beschallung ein 3D-Sensor, vorzugsweise ein Magnet-Sensor, fest mit dem Körper und ein weiterer solcher Sensor mit dem Ultraschallkopf verbunden ist.

6. Verfahren nach einem der Ansprüche 1 bis 5, dadurch gekennzeichnet, daß das optische Bild mit der Darstellung des Datenfeldes am Bildschirm überlagert wird.

7. Verfahren nach einem der Ansprüche 1 bis 6, dadurch gekennzeichnet, daß das optische Bild und die Darstellung des Datenfeldes am Bildschirm in unterschiedlichen Fenstern angezeigt werden.

8. Verfahren nach einem der Ansprüche 1 bis 7, dadurch gekennzeichnet, daß ein Teil des darstellbaren Datenfeldes aus einer willkürlich wählbaren gedachten Achse besteht und daß diese Achse durch folgende Schritte gewonnen wird:
• Darstellung des Körpers als stehendes Bild in mindestens zwei Stellungen;
• Bereitstellung der Möglichkeit durch den Benutzer das Bild der Achse in die stehenden Bilder einzuzeichnen;
• Berechnung der räumlichen Lage der Achse;
• gemeinsame Darstellung der Achse mit dem in Echtzeit beweglich dargestellten Körper.

9. Verfahren nach Anspruch 8, dadurch gekennzeichnet, daß ein Teil des darstellbaren Datenfeldes aus einem willkürlich wählbaren Koordinatensystem besteht, dessen Achsen in der in Anspruch 8 definierten Art gewonnen werden.

10. Vorrichtung zur Darstellung beweglicher Körper, mit der eine optische Darstellung des Körpers und ein dem Körper zugeordnetes Datenfeld darstellbar ist, bestehend aus:
• einer Kamera (1);
• einem Monitor (8), der mit der Kamera in Verbindung steht, um ein von der Kamera aufgenommenes Bild darzustellen; gekennzeichnet durch:
• einem Lagesensor (4), der mit dem darzustellenden Körper (3) fest verbunden ist und der die Bestimmung der jeweiligen Lage des Körpers (3) ermöglicht;
• Mitteln, die mit dem Lagesensor und dem Monitor in Verbindung stehen und die gleichzeitige oder alternative Darstellung des optischen Bildes und des Datenfeldes am Monitor (8) ermöglichen, indem sie fortgesetzt eine optische Darstellung des Datenfeldes berechnen, die am Monitor lagerichtig und in Echtzeit darstellbar ist.

11. Vorrichtung nach Anspruch 10, dadurch gekennzeichnet, daß als Monitor der Bildschirm eines Personal Computers (7) eingesetzt wird, wobei die Kamera (1) am Videoeingang angeschlossen ist und das Datenfeld über den Personal Computer (7) in einen weiteren Eingang eingespielt wird.

## Claims

1. A method for representing movable bodies, wherein an optical representation of the body and a data field associated with the body are displayed, having the following steps:
• Providing an optical imaging system consisting of a camera and a monitor;
• allocation of a spatial data field to the body being disposed in a certain position, in that the imaginary coordinate system of the body and the data field are brought mutually into a relationship so that coincidence is achieved;
• continued detection of the spatial position of the body, comprising both the translational as well as rotary displacement;
• continued calculation of a representation of the data field corresponding to the respective position of the body;
• simultaneous or alternative representation of the optical image and the data field on the monitor, so that the optical represenation of the body and the data field associated with the body are represented simultaneously or alternatively from the same perspective and on the same scale in realtime.

2. A method for representing a human or animal body as claimed in claim 1, characterized in that the position of anatomically characteristic points or a representation gained from an imaging method such as X-ray tomography, NMR tomography or an ultrasonic representation is used as data field.

3. A method as claimed in claim 1 or 2, characterized in that for detecting the position of the body a 3D sensor, preferably a magnetic sensor, is fixedly attached to the body.

4. A method as claimed in claim 3, characterized in that a radiograph is used as data field, whose position to the body is determined in such a way that during the X-ray photography a 3D sensor is fixedly connected to the body and a further such sensor is connected to the X-ray cassette.

5. A method as claimed in claim 3, characterized in that an ultrasonic representation is used as data field, whose position to the body is determined in such a way that during the ultrasonic irradiation a 3D sensor, preferably a magnetic sensor, is fixedly connected to the body and a further such sensor is connected to the ultrasonic head.

6. A method as claimed in one of the claims 1 to 5, characterized in that the optical image is superimposed with the representation of the data field on the screen.

7. A method as claimed in claims 1 to 6, characterized in that the optical image and the representation of the data field on the screen are displayed in different windows.

8. A method as claimed in one of the claims 1 to 7, characterized in that a part of the representable data field consists of a randomly selectable imaginary axis and that said axis is obtained by the following steps:
• Representation of the body as a static image in at least two positions;
• providing the user with the possibility to draw the image of the axis into the static images;
• calculation of the spatial position of the axis;
• joint representation of the axis with the body represented movably in realtime.

9. A method as claimed in claim 8, characterized in that a part of the representable data field consists of a randomly selectable coordinate system whose axes are obtained in the manner as defined in claim 8.

10. An apparatus for representing movable bodies, with which an optical representation of the body and a data field associated with the body is representable, consisting of:
• A camera (1);
• a monitor (8) with which the camera is in connection in order to represent an image recorded by the camera, characterized by:
• a position sensor (4) which is fixedly connected to the body (3) to be represented and allows the definition of the respective position of the body (3);
• means which are in connection with the position sensor and the monitor and allow the simultaneous or alternative representation of the optical image and the data field on the monitor (8) by continuously calculating an optical representation of the data field, which is representable on the monitor in a positionally correct way and in realtime.

11. An apparatus as claimed in claim 10, characterized in that the screen of a personal computer (7) is used as monitor, with the camera (1) being connected to the video input and the data field being delivered via the personal computer (7) to a further input.

## Revendications

1. Procédé pour représenter des corps mobiles, dans lequel sont représentées une représentation visuelle du corps et une zone de données associée au corps, et comprenant les étapes suivantes :
- mise à disposition d'un système de figuration visuelle constitué par une caméra et par un moniteur ;
- association d'une zone de données dans l'espace au corps qui se trouve dans une position déterminée, laquelle est due au fait que les systèmes de coordonnées imaginaires du corps et de la zone de données sont amenés en relation entre eux d'une manière telle que l'on obtienne une coïncidence ;
- poursuite de la détermination de la position du corps dans l'espace, laquelle comprend le déplacement tant en translation qu'en rotation ;
- poursuite du calcul d'une représentation de la zone de données qui correspond à chaque fois à la position du corps ;
- représentation simultanée ou alternative de l'image visuelle et de la zone de données sur le moniteur d'une manière telle que la représentation visuelle du corps et la zone de données qui est associée au corps soient représentées simultanément ou alternativement en temps réel, sous la même perspective et à la même échelle.

2. Procédé pour représenter un corps humain ou animal selon la revendication 1, caractérisé par le fait que l'on utilise comme zone de données la position de points anatomiques caractéristiques ou une représentation issue d'un procédé fournissant une image, comme par exemple une tomographie aux rayons X, une tomographie par résonance magnétique nucléaire ou une représentation au moyen d'ultrasons.

3. Procédé selon l'une des revendications 1 et 2, caractérisé par le fait qu'en vue de la détermination de la position du corps, un capteur fonctionnant sur trois dimensions, lequel est de préférence un capteur magnétique, est relié rigidement au corps.

4. Procédé selon la revendication 3, caractérisé par le fait que l'on utilise comme zone de données une radiographie dont la position par rapport au corps est déterminée grâce au fait que, lors de la prise de vue radiographique, un capteur fonctionnant sur trois dimensions est relié rigidement au corps et un autre capteur de ce genre est relié à la cassette radiographique.

5. Procédé selon la revendication 3, caractérisé par le fait que l'on utilise comme zone de données une représentation par ultrasons dont la position par rapport au corps est déterminée grâce au fait que, lors de l'exposition aux ultrasons, un capteur fonctionnant sur trois dimensions, lequel est de préférence un capteur magnétique, est relié rigidement au corps et un autre capteur de ce genre est relié à la tête qui produit les ultrasons.

6. Procédé selon l'une des revendications 1 à 5, caractérisé par le fait que l'image visuelle et la représentation de la zone de données sont superposées sur l'écran.

7. Procédé selon l'une des revendications 1 à 6, caractérisé par le fait que l'image visuelle et la représentation de la zone de données apparaissent sur l'écran dans des fenêtres différentes.

8. Procédé selon l'une des revendications 1 à 7, caractérisé par le fait qu'une partie de la zone de données qui peut être représentée est constituée par un axe imaginaire que l'on peut choisir arbitrairement, et par le fait que cet axe est obtenu au moyen des étapes suivantes :
- représentation du corps sous la forme d'une image fixe dans deux positions au moins ;
- mise à la disposition de l'utilisateur d'une possibilité de dessiner l'image de l'axe sur les images fixes ;
- calcul de la position de l'axe dans l'espace ;
- représentation commune de l'axe et du corps qui est représenté en temps réel en étant mobile.

9. Procédé selon la revendication 8, caractérisé par le fait qu'une partie de la zone de données qui peut être représentée est constituée par un système de coordonnées, celui-ci pouvant être choisi arbitrairement et ses axes étant obtenus de la manière définie dans la revendication 8.

10. Dispositif pour représenter des corps mobiles au moyen duquel on peut représenter une représentation visuelle du corps et une zone de données associée au corps, constitué par :
- une caméra (1) ;
- un moniteur (8) qui est relié à la caméra pour représenter une image prise par la caméra ;
caractérisé par :
- un capteur de position (4) qui est relié rigidement au corps à représenter (3) et qui permet la détermination de la position correspondante du corps (3) ;
- des moyens qui sont reliés au capteur de position et au moniteur et qui permettent de représenter simultanément ou alternativement sur le moniteur (8) l'image visuelle et la zone de données, du fait qu'ils calculent en continu une représentation visuelle de la zone de données qui peut être représentée sur le moniteur dans sa position vraie et en temps réel.

11. Dispositif selon la revendication 10, caractérisé par le fait que l'on utilise comme moniteur l'écran d'un ordinateur individuel (7), la caméra (1) étant raccordée à l'entrée vidéo et la zone de données étant introduite dans une autre entrée par l'intermédiaire de l'ordinateur individuel (7).
